# EUROPÄISCHE PATENTSCHRIFT

(11) **EP 1 112 720 B1**
(45) Veröffentlichungstag und Bekanntmachung des Hinweises auf die Patenterteilung: **31.08.2005**
(21) Anmeldenummer: 99126142.1
(22) Anmeldetag: 29.12.1999
(51) Int. Cl.: A61B 18/14

(54) **Vorrichtung für die Behandlung von biologischem Gewebe mittels Hochfrequenzstrom**
Device for treating biological tissues with high frequency currents
Appareil pour le traitment des tissues biologiques avec des courants à haute fréquence

(43) Veröffentlichungstag der Anmeldung: 04.07.2001
(73) Patentinhaber: Stockert, Rüdiger, 79104 Freiburg (DE)
(72) Erfinder: Stockert, Rüdiger, 79104 Freiburg (DE)
(74) Vertreter: von Hellfeld, Axel

(56) Entgegenhaltungen:
- EP-A- 0 950 377
- WO-A-96/00036
- WO-A-99/56649
- US-A- 5 577 509
- US-A- 5 906 614
- US-A- 5 957 961

## Beschreibung

Die Erfindung betrifft eine Vorrichtung für die Behandlung von biologischem Gewebe mittels Hochfrequenzstrom.

Derartige Vorrichtungen sind aus der WO 99/07298 bekannt. Sie weisen Elektroden auf, mit denen HF-Strom (Hochfrequenzstrom) in Körpergewebe einkoppelbar ist. Je nach eingestellter Leistung können die Wirkungen des HF-Stromes unterschiedlich sein, z. B. eine Koagulation, eine Schneidwirkung, eine Stimulation oder eine andere angenommene Wirkung, wie Schmerzlinderung bei insbesondere neuralem Gewebe.

Es sind bereits Elektrodensysteme bekannt, bei denen mehrere Elektroden zum Einsatz kommen, entweder mehrere Elektroden auf einem gemeinsamen abstützenden Kopf oder auch in anderer Weise koordiniert eingesetzte Elektroden. Dabei muß jede Elektrode mit HF-Energie versorgt werden. Die eingangs genannte WO 99/07298 beschreibt z. B. mehrere voneinander getrennte Elektrodenflächen auf einem gemeinsamen Kopf, wobei die Elektrodenflächen in unterschiedlicher Struktur angeordnet sein können. Dies ermöglicht einen sehr gezielten Einsatz von HF-Strom entsprechend der jeweils gewünschten Applikation.

Beim Stand der Technik der HF-Behandlungsvorrichtungen mit mehreren Elektroden ist es üblich, einen (einzigen) HF-Generator einzusetzen. Die von dem Hochfrequenzgenerator abgegebene HF-Energie wird wahlweise auf einzelne oder mehrere der Elektroden geschaltet, wofür besondere Schalteinrichtungen erforderlich sind.

Bei diesen elektronischen Schaltungseinrichtungen werden allerdings durch die Schaltfrequenzen starke Störungen an den einzelnen Elektroden erzeugt, wodurch z. B. EKG-Messungen gestört werden können. Auch können diese Störungen eine niederfrequente Potentialableitung an den einzelnen Elektroden unmöglich machen. Auch ist es bei diesem Stand der Technik nicht möglich, die Impedanz an den einzelnen Elektroden kontinuierlich zu messen.

Der Erfindung liegt die Aufgabe zugrunde, eine Vorrichtung für die Behandlung von biologischem Gewebe mittels Hochfrequenzstrom bereitzustellen, mit der vielfältige und genaue Einstellmöglichkeiten hinsichtlich der von einzelnen Elektroden abgegebenen HF-Energie ermöglicht sind.

Erfindungsgemäß wird dieses Ziel durch eine Vorrichtung der eingangs genannten Art erreicht, bei der zwei oder mehrere Hochfrequenzgeneratoren vorgesehen sind, mit denen jeweils HF-Energie an einzelne Elektroden abgebbar ist, wobei die Vorrichtung in Modulbauweise bereitgestellt ist, so daß wahlweise Hochfrequenzgeneratoren zusteckbar bzw. abnehmbar sind, je nach den eingesetzten Elektrodensystemen und der gewünschten Abgabe von HF-Energie.

Dadurch, daß mehr als ein Hochfrequenzgenerator für das Elektrodensystem vorgesehen ist, sind die Möglichkeiten der Ansteuerung der einzelnen Elektroden und ihrer Beschickung mit HF-Energie wesentlich erweitert, so daß in Abhängigkeit von der jeweils gewünschten medizinischen Applikation eine örtliche und/oder zeitliche Optimierung der in das biologische Gewebe eingekoppelten HF-Energie ermöglicht ist.

Gemäß einer bevorzugten Ausgestaltung der Erfindung ist jeweils einer Elektrode aus einer Mehrzahl von Elektroden jeweils ein eigener Hochfrequenzgenerator zugeordnet.

Die Elektroden können dabei entweder zumindest teilweise auf einem sie gemeinsam abstützenden Kopf (vgl. WO 99/07298) angeordnet sein, oder auch auf körperlich getrennten, voneinander unabhängigen Trägern.

Die Erfindung ermöglicht, daß einzelne Hochfrequenzgeneratoren unabhängig voneinander einstellbar sind, insbesondere hinsichtlich ihrer Leistungsabgabe.

US 5,906,614 offenbart eine Vorrichtung zur Behandlung biologischen Gewebes unter Verwendung von Hochfrequenzstrom mit Elektroden. Bei einer Ausführungsform umfasst die Vorrichtung mehrere baueinheitlich integrierte Hochfrequenzgeneratoren, die Elektroden mit Hochfrequenzspannung versorgen, wobei die Energieabgabe in Abhängigkeit der jeweiligen Impedanz des von einem Hochfrequenzgenerators abgegebenen Hochfrequenzstromes erfolgen kann.

US 5,957,961 offenbart eine Vorrichtung für die Behandlung biologischen Gewebes mittels Hochfrequenzstrom, bei der die Energieabgabe in Abhängigkeit von Temperaturen an Elektroden gesteuert wird.

US 5,906,614 offenbart ein System zur Behandlung biologischen Gewebes unter Verwendung von Elektroden, mittels derer dem Gewebe Wärmeenergie zugeführt wird. Das System umfasst einen Generator, der temperaturabhängig gesteuert wird.

WO 99/56640 offenbart, die Energieabgabe eines zur Versorgung mit Elektroden ausgestatteten Katheters vorgesehenem Transformators zu überwachen, um die Impedanz an den Elektroden zu ermitteln.

US 5,577,509 offenbart, über den zwischen zwei, an biologischem Gewebe anliegenden Elektroden fließenden Strom zu erfassen, um elektrische Eigenschaften des Gewebes, insbesondere dessen Impedanz zu ermitteln. Zur Energieversorgung sind die Elektroden mit einem Generator verbunden.

WO 96/00036 offenbart einen mit mehreren Elektroden zur Hochfrequenzbehandlung von biologischem Gewebe ausgestattetem Katheter. Die Steuerung der Energieabgabe über die Elektroden erfolgt in Abhängigkeit von mittels Temperatursensoren erfassten Temperaturen an den Elektroden bzw. diese kontaktierenden Gewebe. Zur Energieversorgung ist ein Generator vorgesehen.

EP 0 950 377 A1 offenbart eine Ablationsanordnung mit mindestens einer Energiequelle. Die mindestens eine Energiequelle ist mit einer Mehrzahl von Energieauskoppelelementen verbunden, um Elektroden, die zur Energiezufuhr zu biologischem Gewebe vorgesehen sind, mit elektrischer Energie zu versorgen. Die Energieauskoppelelemente sind jeweils mit Elektroden eines Katheters über eine Verbindungsleitung verbunden.

Die Erfindung ermöglicht, daß einzelne Hochfrequenzgeneratoren unabhängig voneinander einstellbar sind, insbesondere hinsichtlich ihrer Leistungsabgabe.

Gemäß einer bevorzugten Ausgestaltung der Erfindung ist jedem einzelnen Hochfrequenzgenerator eine "eigene" Steuereinheit zugeordnet, mittels der zumindest ein Parameter des vom zugeordneten Hochfrequenzgenerator abgegebenen Hochfrequenzstroms einstellbar ist. Als einstellbare Parameter kommen insbesondere in Betracht die HF-Energie, die HF-Leistung, die HF-Phase in bezug auf andere Elektroden, die gemessene Impedanz des von der Elektrode beaufschlagten Gewebes, der HF-Strom, die HF-Spannung, die an oder im Bereich dieser Elektrode gemessene Temperatur oder eine andere interessierende Größe.

Gemäß einer weiteren bevorzugten Ausgestaltung der Erfindung ist eine übergeordnete Steuerung vorgesehen, mit der einzelnen HF-Generatoren direkt oder die einzelnen, den jeweiligen Hochfrequenzgeneratoren zugeordneten Steuereinrichtungen wahlweise einzeln, gruppenweise oder insgesamt bezüglich zumindest eines Parameters der von den Hochfrequenzgeneratoren abgegebenen HF-Energie einstellbar sind.

Bevorzugt weisen einzelne Elektroden jeweils eine ihnen zugeordnete Temperaturmeßeinrichtung auf, mit der eine Temperatur meßbar ist, die eine Aussage oder Annahme ermöglicht über die im behandelten Gewebe vorliegende Temperatur. Die Temperaturmeßeinrichtung kann direkt bei, an oder in der Elektrode angeordnet sein.

Gemäß einer anderen bevorzugten Ausgestaltung der Erfindung weisen einzelne Hochfrequenzgeneratoren jeweils eine Meßeinrichtung zum Messen der Impedanz des behandelten Gewebes auf. Alternativ oder zusätzlich können auch Meßeinrichtungen für die abgegebene HF-Leistung, die HF-Spannung, oder die HF-Phase vorgesehen sein, letzteres in bezug auf andere Elektroden.

Gemäß einer bevorzugten Ausgestaltung der Erfindung ist die Vorrichtung so ausgelegt, daß die Ausgänge der einzelnen Hochfrequenzgeneratoren wahlweise oder insgesamt entweder unipolar oder auch bipolar schaltbar sind.

Eine andere bevorzugte Ausgestaltung der Erfindung sieht vor, daß die Gesamtsteuerung und/oder die den einzelnen Hochfrequenzgeneratoren zugeordneten Steuerungen so ausgelegt sind, daß sie die HF-Stromabgabe der einzelnen Hochfrequenzgeneratoren in Abhängigkeit von Eigenschaften oder Messungen benachbarter Elektroden steuern.

Bevorzugt ist vorgesehen, daß alle Hochfrequenzgeneratoren von einem gemeinsamen Bedienfeld ansteuerbar sind. Dabei können bevorzugt über das gemeinsame Bedienfeld Grenzwerte vorgegeben werden, mit denen die Energieabgabe der einzelnen Hochfrequenzgeneratoren wahlweise auf Mindestwerte und/oder Höchstwerte einstellbar ist.

Um die Vorrichtung für EKG (Elektrokardiogramm)-Messungen einsetzbar zu machen, ist gemäß einer weiteren Ausgestaltung vorgesehen, die einzelnen Hochfrequenzgeneratoren jeweils mit bipolaren oder unipolaren EKG-Meßverstärkern auszurüsten.

Eine andere bevorzugte Ausgestaltung der Vorrichtung sieht Steuereinrichtungen vor, mit einem Rechner und einem Datenspeicher, in dem Steuerprogramme ablegbar sind, gemäß denen wahlweise die Leistungsabgabe einzelner Elektroden als Funktion der Zeit steuerbar ist.

In an sich bekannter Weise können die Hochfrequenzgeneratoren so gesteuert werden, daß sie die HF-Energie in gepulster Form oder in amplitudenmodulierter Form abgeben.

Eine andere bevorzugte Ausgestaltung der Erfindung sieht vor, daß im Speicher des Rechners der Steuerung Programme abgelegt sind, mit denen aufgrund abgegebener Hochfrequenzstromleistungen und/oder gemessener Temperaturen eine zu erwartende Temperaturänderung an den Elektroden berechenbar ist, und dass die nachfolgende Hochfrequenzstromabgabe in Abhängigkeit von der berechneten Temperaturentwicklung einstellbar ist.

Zur Vereinfachung der Bedienung ist eine gemeinsame Anzeigeeinrichtung für alle HF-Generatoren und Elektroden geeignet.

Gemäß einer anderen Variante kann die Erfindung kostengünstig auch dadurch realisiert werden, daß ein bestimmter Hochfrequenzgenerator sozusagen als "Master"-Generator fungiert und seine Steuerung auch die Steuerung weiterer hinzufügbarer HF-Generatoren übernimmt.

Eine andere Variante der Vorrichtung sieht elektrische Anschlüsse an der Steuerung bzw. den einzelnen Steuerungen zur Übernahme von Meßergebnissen bezüglich physiologischer Körpersignale an den Elektroden, oder eine Einrichtung zur Messung physiologischer Körpersignale an einzelnen Elektroden vor.

Auch kann für besondere Anwendungen vorgesehen sein, daß Temperatursensoren zwischen einzelnen Elektroden vorgesehen sind und daß die Steuerung bzw. die Steuerungen so ausgelegt sind, daß sie das Temperaturmeßsignal dieser Temperaturfühler auswerten, um zumindest diesem Temperaturfühler benachbarte Elektroden in Abhängigkeit von dem Temperaturmeßergebnis mit Hochfrequenzstrom zu beschicken.

Auch kann es vorteilhaft für die medizinische Applikation sein, gesonderte Elektroden vorzusehen, die nicht für eine Hochfrequenzstromabgabe geschaltet sind und die zwischen hochfrequenzstromabgebenden Elektroden angeordnet sind, wobei mittels der gesonderten Elektroden gemessene physiologische Meßsignale in Steuerungen für die Hochfrequenzgeneratoren übertragen werden, um die Energieabgabe einzelner Elektroden zu steuern.

Schließlich kann die Sicherheit der Vorrichtung und ihre Steuerung dadurch verbessert werden, daß die Elektroden elektronische Identifikationsträger (Chips) aufweisen, die Daten enthalten über die jeweilige Elektrode und die von einer Steuerung der Vorrichtung ausgelesen werden können.

Gemäß einer weiteren bevorzugten Ausgestaltung der Erfindung ist vorgesehen, daß die relative Phasenlage der einzelnen HF-Generatoren zueinander einstell- und insbesondere veränderbar ist. Mit dieser Variante der Erfindung ist es möglich, Additionseffekte bzw. Subtraktionseffekte bezüglich der HF-Energie auf die einzelnen Elektroden zu beeinflussen.

Gemäß einer weiteren Variante der Erfindung ist vorgesehen, daß die HF-Generatoren teilweise oder alle mit unterschiedlichen Frequenzen arbeiten. So kann z. B. vorgesehen sein, daß in der Reihe von Elektroden abwechselnd HF-Energie mit den Frequenzen 470, 490, 470, 490 etc. kHz abgegeben werden. Ebenfalls möglich ist eine unterschiedliche Einstellung der Frequenzen mit kleinen Stufen etwa wie folgt: 430, 450, 470, 490 etc. kHz. Durch unterschiedliche Frequenzen der einzelnen HF-Generatoren ist es möglich, meßtechnische Schwierigkeiten zu überwinden, wie etwa die Rückwirkung auf benachbarte Generatoren oder Phasenprobleme, Amplitudenmeßprobleme etc.

Auch bei der Impedanzmessung können Wechselwirkungen zwischen den einzelnen Meßkreisen durch verschiedene Frequenzen vermieden werden, z. B. kann folgende Frequenzverteilung vorgesehen sein: 50 kHz, 51 kHz, 50 kHz, 51 kHz etc. Ebenfalls möglich ist z. B. folgende Frequenzverteilung: 50 kHz, 51 kHz, 52 kHz, 53 kHz, 54 kHz etc.

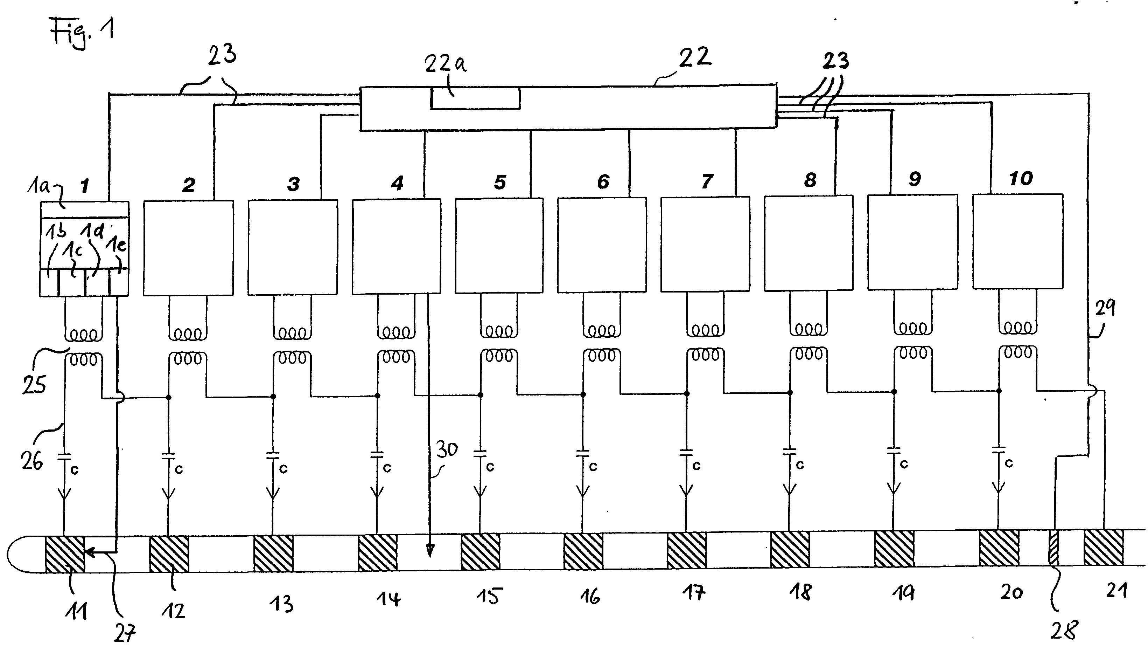

Nachfolgend werden Ausführungsbeispiele der Erfindung anhand der Zeichnungen näher erläutert. Es zeigt:
- Figur 1: schematisch eine Vorrichtung für die Behandlung von biologischem Gewebe mittels Hochfrequenzstrom mit bipolarer Schaltung der Elektroden; und
- Figur 2: eine Vorrichtung für die Behandlung von biologischem Gewebe mittels Hochfrequenzstrom mit Elektroden in unipolarer Schaltung.
Beim Ausführungsbeispiel einer Vorrichtung für die Hochfrequenzbehandlung von Körpergewebe gemäß Figur 1 sind 10 HF-Generatoren 1, 2, 3, 4, 5, 6, 7, 8, 9 und 10 nebeneinander angeordnet. Die Hochfrequenzgeneratoren und die weiter unten näher beschriebenen zugehörigen Steuereinrichtungen sind in einem gemeinsamen Gehäuse bzw. auf einer gemeinsamen Abstützung angeordnet. Es kann auch vorgesehen sein, die einzelnen HF-Generatoren mit zugehörigen Bau-, Steuer- und Meßeinrichtungen in Modulbauweise so auszuführen, daß wahlweise zwei und mehr HF-Generatoren zu einem Gerät zusammenfügbar sind.

Jeder der HF-Generatoren 1, 2, ... 10 ist mit einer gesonderten, ihm (und nur ihm) zugeordneten Elektrode 11, 12, 13, 14, 15, 16, 17, 18, 19 und 20 verbindbar. Bei der bipolaren Schaltung gemäß Figur 1 ist eine weitere Elektrode 21 als Bezugselektrode vorgesehen.

Die in Figur 1 mit den Bezugszeichen 1, 2, 3, ..., 10 versehenen Funktionsblöcke enthalten außer den eigentlichen HF-Generatoren noch deren jeweilige Steuer- und Meßverstärker, wie weiter unten näher erläutert wird.

Beim dargestellten Ausführungsbeispiel gemäß Figur 1 ist über die individuellen Steuereinrichtungen für die einzelnen HF-Generatoren hinaus noch eine Gesamtsteuerung 22 vorgesehen. Die Gesamtsteuerung 22 ist über Leitungen 23, die auch als Bus ausgebildet sein können, mit den einzelnen Hochfrequenzgeneratoren 1, 2, ..., 10 einschließlich deren jeweiligen Steuerungen verbunden. Die jeweiligen individuellen Steuerungen für die einzelnen HF-Generatoren 1, 2, ..., 10 sind in Figur 1 beispielhaft beim HF-Generator 1 mit dem Bezugszeichen la gezeigt und bezeichnet.

Über Ausgangstransformatoren 25 sind die einzelnen HF-Generatoren jeweils mit ihrer zugeordneten Elektrode verbunden, wobei in Figur 1 nur eine Leitung beispielhaft mit dem Bezugszeichen 26 gekennzeichnet ist.

Die Ausgänge der einzelnen HF-Generatoren (also die elektrodenseitigen Ausgänge der Ausgangstransformatoren 25) sind so gestaltet, daß die einzelnen Elektroden wahlweise von "unipolar" auf "bipolar" und umgekehrt umgeschaltet werden können. Bei unipolaren Einsätzen werden alle Ausgangswindungen auf einer Seite mit der gemeinsamen Elektrode 31 verbunden, deren Potential frei ist. Die andere Seite der Ausgangswindungen wird, wie in den Figuren gezeigt ist, über den Entkopplungskondensator C mit der jeweiligen Elektrode verbunden. Bei der bipolaren Schaltung werden hingegen alle Ausgangsspulen der Ausgangstransformatoren (HF-Übertrager) mit jeweils zwei Elektroden verbunden, bei dargestellten Ausführungsbeispiel gemäß Figur 1 sind dies benachbarte Elektroden. Dies bedeutet, daß ein Hochfrequenzgenerator immer auf zwei Elektroden wirkt.

Figur 1 zeigt weiter einen Temperatursensor 27, der der ersten Elektrode 11 zugeordnet ist. Die anderen Elektroden können entsprechende Temperatursensoren (nicht gezeigt) aufweisen. Beispiele, wie die Temperatursensoren im einzelnen ausgeführt sein können, sind im oben zitierten Stand der Technik genannt. Das Meßsignal des Temperatursensors 27 wird in einen Temperatur-Meßverstärker le übertragen, der mit der Steuerung 1a dieses HF-Generators verbunden ist. Weiterhin weist der HF-Generator 1 eine Meßeinrichtung 1b für die Phase des abgegebenen HF-Stromes, eine Meßeinrichtung lc für die Impedanz des mit Strom beschickten Gewebes und eine Meßeinrichtung 1d für die abgegebene HF-Leistung auf. Diese Meßeinrichtungen sind Teil der zugeordneten Steuerung la. Entsprechendes gilt für die anderen HF-Generatoren (in der Figur nicht im einzelnen wiederholt).

Figur 1 zeigt weiterhin beispielhaft eine weitere Elektrode 28, die hier der Messung einer physiologischen Größe dient (also nicht für die HF-Abgabe). Das Meßsignal der Elektrode 28 wird über eine Leitung 29 in die Gesamtsteuerung 22 eingegeben und kann dort mittels abgelegter Rechenprogramme in Steuersignale für die einzelnen HF-Generatoren umgesetzt werden.

Weiterhin zeigt Figur 1 beispielhaft und schematisch einen Temperatursensor 30, der - anders als der Temperatursensor 27 - zwischen zwei Elektroden (hier 14 und 15) angeordnet ist. Entsprechend können zwischen anderen Elektroden weitere Temperatursensoren (nicht gezeigt) angeordnet sein. Das Meßsignal des Temperatursensors 30 wird in die Steuerung des zugeordneten HF-Generators (hier der HF-Generator 4) eingegeben oder in die Gesamtsteuerung 22.

Die in Figur 1 schematisch dargestellten Elektroden 11, 12, 13, ..., 21 können auf einem einzigen Träger angeordnet sein (analog etwa der WO 99/07298) oder auch auf physisch getrennten Elektrodenträgern (Köpfen).

Jeder der HF-Generatoren 1, 2, ..., 10 kann mit einer Anzeigeeinrichtung versehen sein, insbesondere bezüglich der gemessenen Temperatur und der augenblicklich abgegebenen Leistung oder bezüglich der gemessenen Impedanz. Auch kann eine vorgewählte Energie anzeigbar sein.

In diesem Sinne ist beim dargestellten Ausführungsbeispiel gemäß Figur 1 jeder der einzelnen HF-Generatoren jeweils eine autarke Einheit derart, daß mit dieser Einheit eine individuelle und vollständige Steuerung der jeweils zugeordneten Elektrode möglich ist.

Darüber hinaus zeigt Figur 1 mit der Gesamtsteuerung 22 und deren Bedienfeld 22a die Möglichkeit, die einzelnen HF-Generatoren und deren Steuerungen von einer zentralen Stelle aus zu steuern. Die HF-Frequenz kann zwischen 200 kHz und 2 MHz eingestellt werden. Für die Impedanzmessung kann eine mittelfrequente Sinusschwingung vorgesehen werden zwischen 50 und 200 kHz.

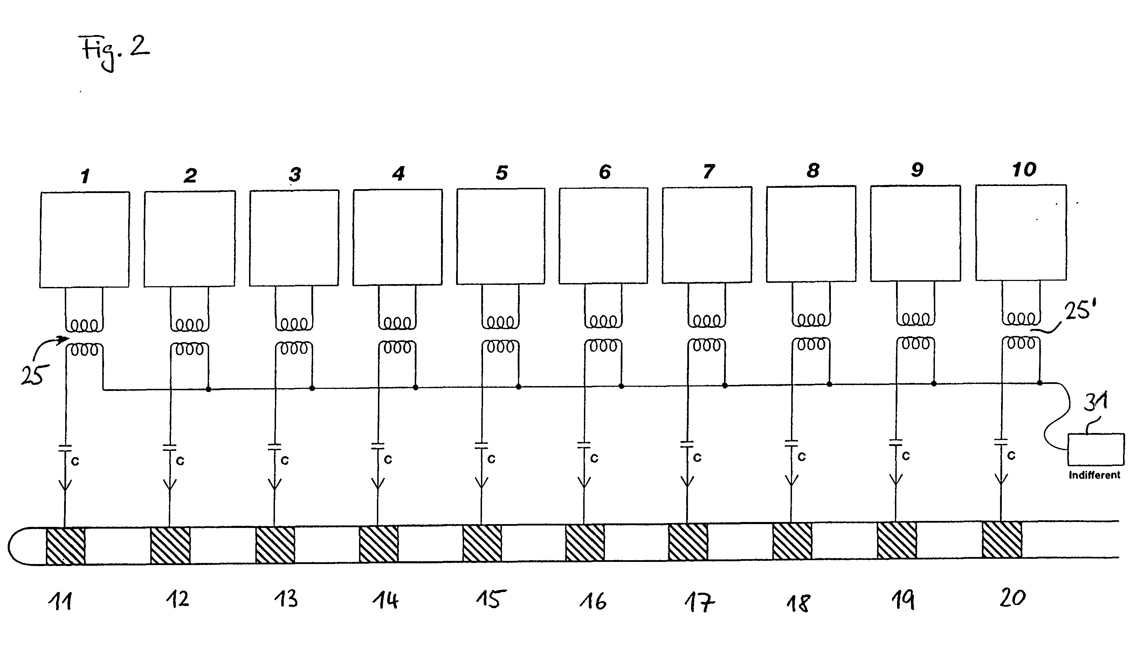

Figur 2 zeigt eine Vorrichtung für die Behandlung von biologischem Gewebe mittels Hochfrequenzstrom entsprechend Figur 1. Der Unterschied liegt in der Schaltung der Elektroden: Während bei Figur 1 die Elektroden bipolar geschaltet sind, ist die Schaltung der Elektroden gemäß Figur 2 unipolar, wozu ein indifferentes Potential 31 vorgesehen ist, das das Bezugspotential für alle Ausgangstransformatoren 25 bildet. Ansonsten enthält die Vorrichtung gemäß Figur 2 sämtliche Komponenten, die in Figur 1 gezeigt und oben beschrieben sind, und die in Figur 2 nicht noch einmal wiederholt sind.

Durch den beschriebenen statischen Aufbau und Betrieb der oben beschriebenen Vorrichtungen gibt es keine Potentialverschiebung an den einzelnen Elektrodenflächen. Deshalb ist eine Messung von physiologischen Signalen an den Elektroden möglich. Durch eine insbesondere vorgesehene kontinuierliche Impedanzmessung an jeder einzelnen Einheit kann eine "Echtzeit"-Impedanzdarstellung auf einem Anzeigegerät vorgenommen werden. Weiterhin ist bevorzugt vorgesehen, die Temperatur kontinuierlich zu messen und aktuell anzuzeigen, so daß der Benutzer sofort den Temperaturverlauf bei jeder Elektrode erkennt. Durch das für alle Elektroden vorgesehene gemeinsame Bedienungsfeld 22a können alle Generatoren gleichzeitig gesteuert werden, d. h. an diesem Bedienungsfeld können die interessierenden Parameter der einzelnen HF-Generatoren eingestellt werden. Beispielsweise kann die maximale Leistung jedes einzelnen Generators sowie die Gesamtleistung des gesamten Systems (aller Generatoren) limitiert werden, entsprechendes gilt auch für die Temperaturen und die gemessenen Impedanzen.

Insbesondere ist es möglich, sog. Tendenzen anzuzeigen, d. h. das dynamische Verhalten einzelner gemessener oder berechneter Parameter grafisch auf einem Anzeigegerät darzustellen. Damit kann ein Benutzer beispielsweise zu schnelle Temperatur- oder Impedanzanstiege erkennen und beeinflussen.

## Patentansprüche

1. Vorrichtung für die Behandlung von biologischem Gewebe mittels Hochfrequenzstrom mit Elektroden (11, 12, ... , 21), mit zwei oder mehr Hochfrequenzgeneratoren (1, 2, ... 10), zur Versorgung von Elektroden mit Hochfrequenzspannung, **gekennzeichnet durch** eine Modulbauweise, gemäß der wahlweise Hochfrequenzgeneratoren zusteckbar bzw. abnehmbar sind.

2. Vorrichtung nach Anspruch 1,
**dadurch gekennzeichnet, dass**
jeweils einer Elektrode (z.B. 11) aus einer Mehrzahl von Elektroden (11 ... 21) jeweils ein Hochfrequenzgenerator zugeordnet ist.

3. Vorrichtung nach einem der Ansprüche 1 oder 2,
**dadurch gekennzeichnet, dass**
einzelne Hochfrequenzgeneratoren (1, 2, 3, ..., 10) unabhängig voneinander einstellbar sind, insbesondere hinsichtlich ihrer Leistungsabgabe.

4. Vorrichtung nach einem der vorhergehenden Ansprüche, **dadurch gekennzeichnet, dass**
jedem einzelnen Hochfrequenzgenerator eine Steuereinheit (1a) zugeordnet ist, mittels der zumindest ein Parameter des vom zugeordneten Hochfrequenzgenerator abgegebenen Hochfrequenzstromes einstellbar ist.

5. Vorrichtung nach Anspruch 4,
**dadurch gekennzeichnet, dass**
eine übergeordnete Steuerung (22) vorgesehen ist, mit der die einzelnen Steuereinrichtungen (1a) für die Hochfrequenzgeneratoren wahlweise einzeln, gruppenweise oder insgesamt bezüglich zumindest eines Parameters des von den Hochfrequenzgeneratoren abgegebenen Stromes und/oder der Spannung einstellbar sind.

6. Vorrichtung nach einem der vorhergehenden Ansprüche,
**dadurch gekennzeichnet, dass**
einzelne Elektroden jeweils eine Temperaturmesseinrichtung (27) aufweisen, und dass das Temperaturmessergebnis der Messeinrichtung zu der zugeordneten Steuereinheit dieser Elektrode übertragen wird.

7. Vorrichtung nach einem der vorhergehenden Ansprüche,
**dadurch gekennzeichnet, dass**
den einzelnen Hochfrequenzgeneratoren jeweils eine Impedanzmesseinrichtung zugeordnet ist.

8. Vorrichtung nach einem der vorhergehenden Ansprüche,
**dadurch gekennzeichnet, dass**
den einzelnen Hochfrequenzgeneratoren jeweils eine Leistungsmesseinrichtung (1d) zugeordnet ist.

9. Vorrichtung nach einem der vorhergehenden Ansprüche,
**dadurch gekennzeichnet, dass**
die einzelnen Hochfrequenzgeneratoren jeweils Messeinrichtungen (1b) zum Messen der Hochfrequenzspannung, des Hochfrequenzstromes und/oder der Hochfrequenzphase aufweisen.

10. Vorrichtung nach einem der vorhergehenden Ansprüche,
**dadurch gekennzeichnet, dass**
die Ausgänge der einzelnen Hochfrequenzgeneratoren so ausgelegt sind, dass die Elektroden sowohl unipolar als auch bipolar schaltbar sind.

11. Vorrichtung nach einem der vorhergehenden Ansprüche,
**dadurch gekennzeichnet, dass**
die Steuerungen (1a) für die einzelnen Hochfrequenzgeneratoren so ausgelegt sind, dass sie die Hochfrequenzstromabgabe ihres zugeordneten Hochfrequenzgenerators in Abhängigkeit von Eigenschaften oder Messgrößen benachbarter Elektroden steuern.

12. Vorrichtung nach einem der vorhergehenden Ansprüche, **dadurch gekennzeichnet, dass**
alle Hochfrequenzgeneratoren von einem gemeinsamen Bedienfeld (22a) ansteuerbar sind.

13. Vorrichtung nach Anspruch 12,
**dadurch gekennzeichnet, dass**
mittels des allen Hochfrequenzgeneratoren gemeinsamen Bedienfeldes (22a) Grenzwerte einstellbar sind, mit denen die Energieabgabe der einzelnen Hochfrequenzgeneratoren wahlweise auf Mindestwerte und/oder Höchstwerte einstellbar ist.

14. Vorrichtung nach einem der vorhergehenden Ansprüche,
**dadurch gekennzeichnet, dass**
einzelne Hochfrequenzgeneratoren jeweils über eigene bipolare oder unipolare Ekg-Messverstärker verfügen.

15. Vorrichtung nach Anspruch 4
**dadurch gekennzeichnet, dass**
den einzelnen Steuereinrichtungen für die Hochfrequenzgeneratoren jeweils ein oder mehrere Temperaturmessverstärker (1e) zugeordnet ist bzw. sind.

16. Vorrichtung nach einem der vorhergehenden Ansprüche,
**dadurch gekennzeichnet, dass**
eine Steuereinrichtung (22) vorgesehen ist mit einem Rechner und einem Speicher, in dem Steuerprogramme abgelegt sind, gemäß denen wahlweise die Leistungsabgabe jeder einzelnen Elektrode und/oder die Leistungsabgabe einzelner ausgewählter Elektroden und/oder die Leistungsabgabe aller Elektroden als Funktion der Zeit steuerbar ist.

17. Vorrichtung nach einem der vorhergehenden Ansprüche,
**dadurch gekennzeichnet, dass**
die Steuerungen der Hochfrequenzgeneratoren ermöglichen, dass die Elektroden den Hochfrequenzstrom in gepulster Form oder in amplitudenmodulierter Form abgeben.

18. Vorrichtung nach Anspruch 15
**dadurch gekennzeichnet, dass**
die Abgabe von Hochfrequenzstrom durch die Hochfrequenzgeneratoren in Abhängigkeit von an den Elektroden gemessenen Temperaturen regelbar ist, insbesondere auf Temperaturen in einem Einstellbereich von 20°C bis 120°C.

19. Vorrichtung nach Anspruch 16
**dadurch gekennzeichnet, dass**
im Speicher des Rechners der Steuerung Programme abgelegt sind, mit denen aufgrund abgegebener Hochfrequenzstromleistungen und/oder gemessener Temperaturen eine zu erwartende Temperaturänderung an den Elektroden zu berechnen ist, und dass die nachfolgende Hochfrequenzstromabgabe in Abhängigkeit von der berechneten Temperaturentwicklung einstellbar ist.

20. Vorrichtung nach einem der vorhergehenden Ansprüche,
**dadurch gekennzeichnet, dass**
Einrichtungen vorgesehen sind zum Messen der Impedanz bezüglich des von einzelnen Hochfrequenzgeneratoren oder einer Gruppe oder allen Hochfrequenzgeneratoren abgegebenen Hochfrequenzstromes und dass mittels einer Steuerung die Energieabgabe einzelner Generatoren, einer Gruppe von Generatoren oder aller Generatoren in Abhängigkeit von der gemessenen Impedanz geregelt wird.

21. Vorrichtung nach einem der vorhergehenden Ansprüche, **gekennzeichnet durch** eine gemeinsame Anzeigeeinrichtung.

22. Vorrichtung nach einem der vorhergehenden Ansprüche,
**dadurch gekennzeichnet, dass**
mittels eines Hochfrequenzgenerators und seiner Steuereinrichtung andere Hochfrequenzgeneratoren steuer- und einstellbar sind.

23. Vorrichtung nach Anspruch 4,
**gekennzeichnet durch**
elektrische Anschlüsse an der Steuerung bzw. den einzelnen Steuerungen zur Übernahme von Messergebnissen bezüglich physiologischer Körpersignale an den Elektroden, oder eine Einrichtung zur Messung physiologischer Körpersignale an einzelnen Elektroden.

24. Vorrichtung nach einem der vorhergehenden Ansprüche,
**dadurch gekennzeichnet, dass**
Temperatursensoren (30) zwischen einzelnen Elektroden vorgesehen sind und dass die Steuerung bzw. die Steuerungen so ausgelegt sind, dass sie das Temperaturmesssignal dieser Temperaturfühler auswerten, um zumindest diesem Temperaturfühler benachbarte Elektroden in Abhängigkeit von dem Temperaturmessergebnis mit Hochfrequenzstrom zu beschicken.

25. Vorrichtung nach einem der vorhergehenden Ansprüche, **gekennzeichnet durch**
zumindest eine gesonderte Elektrode (28), die nicht für eine Hochfrequenzstromabgabe geschaltet und die zwischen hochfrequenzstromabgebenden Elektroden angeordnet ist, wobei mittels der gesonderten Elektrode gemessene physiologische Messsignale in Steuerungen für die Hochfrequenzgeneratoren übertragbar sind, um die Energieabgabe einzelner Elektroden zu steuern.

26. Vorrichtung nach einem der vorhergehenden Ansprüche,
**dadurch gekennzeichnet, dass**
die Hochfrequenzgeneratoren über ein Bus-System miteinander gekoppelt sind.

27. Vorrichtung nach einem der vorhergehenden Ansprüche,
**dadurch gekennzeichnet, dass**
die Elektroden elektronische Identifikationsträger (Chips) aufweisen, die Daten enthalten über die jeweilige Elektrode und die von einer Steuerung der Vorrichtung ausgelesen werden können.

28. Vorrichtung nach einem der vorhergehenden Ansprüche,
**dadurch gekennzeichnet, dass**
die Phasenlage der einzelnen Hochfrequenzgeneratoren (1, 2, ... 10) relativ zueinander einstellbar ist.

29. Vorrichtung nach einem der vorhergehenden Ansprüche,
**dadurch gekennzeichnet, dass**
zumindest ein Teil der Hochfrequenzgeneratoren mit unterschiedlichen Frequenzen Hochfrequenzenergie abgibt.

## Claims

1. Device for treatment of biological tissue by means of high-frequency current with electrodes (11, 12, ..., 21), with two or more high-frequency generators (1, 2, ..., 10), for supplying electrodes with high-frequency voltage, **characterised by** a modular design according to which high-frequency generators can optionally be added or removed.

2. Device according to claim 1, **characterised in that** in each case one electrode (e.g. 11) of a plurality of electrodes (11 ... 21) has associated therewith a high-frequency generator.

3. Device according to one of claims 1 or 2, **characterised in that** individual high-frequency generators (1, 2, 3, ..., 10) are adjustable independently of one another, in particular with regard to their power delivery.

4. Device according to one of the preceding claims, **characterised in that** each individual high-frequency generator has associated therewith a control unit (1a) by means of which at least one parameter of the high-frequency current delivered by the associated high-frequency generator can be adjusted.

5. Device according to claim 4, **characterised in that** a master controller (22) is provided with which the individual control units (1a) for the high-frequency generators can optionally be adjusted individually, in groups or as a whole with regard to at least one parameter of the current and/or voltage delivered by the high-frequency generators.

6. Device according to one of the preceding claims, **characterised in that** individual electrodes each exhibit a temperature measuring device (27), and **in that** the temperature measurement result of the measuring device is transmitted to the associated control unit of this electrode.

7. Device according to one of the preceding claims, **characterised in that** the individual high-frequency generators each have associated therewith an impedance measuring device.

8. Device according to one of the preceding claims, **characterised in that** the individual high-frequency generators each have associated therewith a power measuring device (1d).

9. Device according to one of the preceding claims, **characterised in that** the individual high-frequency generators each exhibit measuring devices (1b) for measuring the high-frequency voltage, the high-frequency current and/or the high-frequency phase.

10. Device according to one of the preceding claims, **characterised in that** the outputs of the individual high-frequency generators are designed so that the electrodes can be connected both in unipolar and in bipolar mode.

11. Device according to one of the preceding claims, **characterised in that** the control units (1a) for the individual high-frequency generators are designed so that they control the delivery of high-frequency current of their associated high-frequency generator according to properties or measured variables of neighbouring electrodes.

12. Device according to one of the preceding claims, **characterised in that** all the high-frequency generators can be actuated by a common control panel (22a).

13. Device according to claim 12, **characterised in that** the control panel (22a) common to all the high-frequency generators can be used to set limit values with which the energy delivery of the individual high-frequency generators can optionally be adjusted to minimum values and/or maximum values.

14. Device according to one of the preceding claims, **characterised in that** individual high-frequency generators each have their own bipolar or unipolar ECG measuring amplifiers.

15. Device according to claim 4, **characterised in that** the individual control units for the high-frequency generators each have associated therewith one or more temperature measuring amplifiers (1e).

16. Device according to one of the preceding claims, **characterised in that** a controller (22) is provided with a computer and a memory in which control programmes are stored according to which the power delivery of each individual electrode and/or the power delivery of individual selected electrodes and/or the power delivery of all the electrodes can be controlled as a function of the time.

17. Device according to one of the preceding claims, **characterised in that** the control units of the high-frequency generators allow the electrodes to deliver the high-frequency current in pulsed form or in amplitude-modulated form.

18. Device according to claim 15, **characterised in that** the delivery of high-frequency current by the high-frequency generators can be regulated depending on temperatures measured at the electrodes, in particular temperatures in a range of adjustment from 20°C to 120°C.

19. Device according to claim 16, **characterised in that** the memory of the computer of the controller holds programmes with which an expected temperature change at the electrodes can be calculated on the basis of delivered high-frequency current outputs and/or measured temperatures, and **in that** the following high-frequency current delivery can be adjusted according to the calculated temperature trend.

20. Device according to one of the preceding claims, **characterised in that** devices are provided for measurement of the impedance with respect to the high-frequency current delivered by individual high-frequency generators or a group or all of the high-frequency generators, and **in that** the energy delivery of individual generators, a group of generators or all of the generators is regulated according to the measured impedance by means of a controller.

21. Device according to one of the preceding claims, **characterised by** a common indicating device.

22. Device according to one of the preceding claims, **characterised in that** other high-frequency generators can be controlled and adjusted by means of a high-frequency generator and its control unit.

23. Device according to claim 4, **characterised by** electrical connections to the controller or the individual control units for transmission of measurement results with respect to physiological body signals at the electrodes, or a device for measurement of physiological body signals at individual electrodes.

24. Device according to one of the preceding claims, **characterised in that** temperature sensors (30) are provided between individual electrodes and **in that** the controller or control units are designed so that they evaluate the temperature measurement signal of these temperature sensors in order to supply at least electrodes neighbouring this temperature sensor with high-frequency current according to the temperature measurement result.

25. Device according to one of the preceding claims, **characterised by** at least one separate electrode (28) which is not connected for delivering high-frequency current and which is arranged between electrodes delivering high-frequency current, whereby physiological measurement signals measured by means of the separate electrode can be transmitted to control units for the high-frequency generators in order to control the energy delivery of individual electrodes.

26. Device according to one of the preceding claims, **characterised in that** the high-frequency generators are coupled to one another by means of a bus system.

27. Device according to one of the preceding claims, **characterised in that** the electrodes exhibit electronic identification carriers (chips) which contain data which relate to the respective electrode and which can be read by a controller of the device.

28. Device according to one of the preceding claims, **characterised in that** the phase position of the individual high-frequency generators (1, 2, ..., 10) can be adjusted relative to one another.

29. Device according to one of the preceding claims, **characterised in that** at least one part of the high-frequency generators delivers high-frequency energy with different frequencies.

## Revendications

1. Dispositif pour le traitement d'un tissu biologique à l'aide d'un courant à haute fréquence avec des électrodes (11,12,...,21), comportant deux ou plus de deux générateurs de haute fréquence (1,2,...10) pour alimenter des électrodes avec une tension à haute fréquence, **caractérisé par**
une construction modulaire, sur la base de laquelle on peut connecter ou retirer au choix des générateurs de haute fréquence.

2. Dispositif selon la revendication 1, **caractérisé en ce qu'**à une électrode respective (par exemple 11) constituée par une multiplicité d'électrodes (11;21) est associé respectivement un générateur de haute fréquence.

3. Dispositif selon l'une des revendications 1 ou 2, **caractérisé en ce que** des générateurs individuels de haute fréquence (1,2,3,....,10) sont réglables indépendamment les uns des autres, notamment en ce qui concerne la puissance qu'ils délivrent.

4. Dispositif selon l'une des revendications précédentes, **caractérisé en ce qu'**à chaque générateur individuel de haute fréquence est associée une unité de commande (1a), à l'aide de laquelle au moins un paramètre du courant à haute fréquence délivré par le générateur de haute fréquence associé, est réglable.

5. Dispositif selon la revendication 4, **caractérisé en ce qu'**il est prévu une unité de commande de rang supérieur (22), au moyen de laquelle les différents dispositifs de commande (1a) pour les générateurs de haute fréquence sont réglables au choix individuellement, selon des groupes ou globalement en rapport avec au moins un paramètre du courant délivré par les générateurs de haute fréquence et/ou de la tension.

6. Dispositif selon l'une des revendications précédentes, **caractérisé en ce que** des électrodes individuelles possèdent respectivement un dispositif de mesure de température (27) et que le résultat de mesure de température du dispositif de mesure est transmis à l'unité de commande associée de cette électrode.

7. Dispositif selon l'une des revendications précédentes, **caractérisé en ce que** respectivement un dispositif de mesure d'impédance est associé aux différents générateurs de haute fréquence.

8. Dispositif selon l'une des revendications précédentes, **caractérisé en ce que** respectivement un dispositif de mesure de puissance (1d) est associé aux différents générateurs de haute fréquence.

9. Dispositif selon l'une des revendications précédentes, **caractérisé en ce que** les différents générateurs de haute fréquence comportent respectivement des dispositifs de mesure (1b) pour la mesure de la tension à haute fréquence, du courant à haute fréquence et/ou de la phase à haute fréquence.

10. Dispositif selon l'une des revendications précédentes, **caractérisé en ce que** les sorties des différents générateurs de haute fréquence sont conçues de telle sorte que les électrodes peuvent être commutées aussi bien de façon unipolaire que de façon bipolaire.

11. Dispositif selon l'une des revendications précédente, **caractérisé en ce que** les unités de commande (1a) pour les différents générateurs de haute fréquence sont conçues de telle sorte qu'elles commandent la délivrance d'un courant à haute fréquence du générateur de haute fréquence, qui leur est associé, en fonction de caractéristiques ou de grandeurs de mesure d'électrodes voisines.

12. Dispositif selon l'une des revendications précédente, **caractérisé en ce que** tous les générateurs de haute fréquence peuvent être commandés par un panneau de commande commun (22a).

13. Dispositif selon la revendication 12, **caracté** **risé en ce qu'**à l'aide du panneau de commande (22a), qui est commun à tous les générateurs de haute fréquence, on peut régler des valeurs limites, avec lesquelles la délivrance d'énergie des différents générateurs de haute fréquence est réglable au choix sur des valeurs minimales et/ou sur des valeurs maximales.

14. Dispositif selon l'une des revendications précédente, **caractérisé en ce que** différents générateurs de haute fréquence disposent respectivement d'un amplificateur bipolaire ou unipolaire propre de mesure d'électrocardiogrammes.

15. Dispositif selon la revendication 4, **caractérisé en ce qu'**aux différents dispositifs de commande pour les générateurs de haute fréquence sont associés respectivement un ou plusieurs amplificateurs de mesure de température (1a).

16. Dispositif selon l'une des revendications précédente, **caractérisé en ce qu'**il est prévu un dispositif de commande (22) comportant un calculateur et une mémoire, dans laquelle sont mémorisés des programmes de commande, conformément auxquels au choix la délivrance de puissance de chaque électrode individuelle et/ou la délivrance de puissance d'électrodes individuelles sélectionnées et/ou la délivrance de puissance de toutes les électrodes peut être commandée en fonction du temps.

17. Dispositif selon l'une des revendications précédente, **caractérisé en ce que** les unités de commande des générateurs à haute fréquence permettent que les électrodes délivrent le courant à haute fréquence sous forme pulsée ou sous une forme modulée en amplitude.

18. Dispositif selon la revendication 15, **caractérisé en ce que** la délivrance d'un courant à haute fréquence par les générateurs de haute fréquence est réglable en fonction de températures mesurées au niveau des électrodes, notamment de températures dans une plage de réglage de 20°C à 120°C.

19. Dispositif selon la revendication 16, **caractérisé en ce que** dans la mémoire du calculateur de l'unité de commande sont mémorisés des programmes, à l'aide desquels une variation de température, à laquelle on peut s'attendre, au niveau des électrodes, doit être calculée sur la base de puissances délivrées du courant à haute fréquence et/ou de températures mesurées, et que la délivrance ultérieure du courant à haute fréquence est réglable en fonction du développement de température calculé.

20. Dispositif selon l'une des revendications précédente, **caractérisé en ce que** des dispositifs sont prévus pour mesurer l'impédance en rapport avec le courant à haute fréquence délivré par des générateurs individuels de haute fréquence ou par un groupe ou par la totalité des générateurs de haute fréquence, et que la délivrance d'énergie de générateurs individuels, d'un groupe de générateurs ou de tous les générateurs est réglée en fonction de l'impédance mesurée, au moyen d'une unité de commande.

21. Dispositif selon l'une des revendications précédente, **caractérisé par** un dispositif d'affichage commun.

22. Dispositif selon l'une des revendications précédente, **caractérisé en ce qu'**à l'aide d'un générateur de haute fréquence et de son dispositif de commande, il est possible de commander et de régler d'autres générateurs de haute fréquence.

23. Dispositif selon la revendication 4, **caractérisé par** des connexions électriques avec l'unité de commande ou les différentes unités de commande pour le transfert de résultats de mesure concernant des signaux corporels physiologiques aux électrodes, ou un dispositif pour mesurer des signaux corporels physiologiques au niveau d'électrodes individuelles.

24. Dispositif selon l'une des revendications précédente, **caractérisé en ce que** des capteurs de température (30) sont prévus entre différentes électrodes et que l'unité de commande ou les unités de commande sont agencées de telle sorte qu'elles évaluent le signal de mesure de température de ces capteurs de température, pour envoyer un courant à haute fréquence aux électrodes voisines au moins de ce capteur de température, en fonction du résultat de mesure de la température.

25. Dispositif selon l'une des revendications précédente, **caractérisé par**
au moins une électrode particulière (28), qui n'est pas branchée pour une délivrance du courant à haute fréquence et qui est disposée entre des électrodes délivrant un courant de fréquence, des signaux de mesure physiologiques mesurés à l'aide de l'électrode particulière pouvant être transmis dans des unités de commande pour les générateurs de haute fréquence, de manière à commander la délivrance d'énergie à des électrodes individuelles.

26. Dispositif selon l'une des revendications précédente, **caractérisé en ce que** les générateurs de haute fréquence sont couplés entre eux par l'intermédiaire d'un système de bus.

27. Dispositif selon l'une des revendications précédente, **caractérisé en ce que** les électrodes comportent des supports électroniques d'identification (chips), qui contiennent des données concernant l'électrode respective et qui peuvent être lus par l'unité de commande du dispositif.

28. Dispositif selon l'une des revendications précédente, **caractérisé en ce que** la position de phase réciproque des différents générateurs de haute fréquence (1,2,...10) est réglable.

29. Dispositif selon l'une des revendications précédente, **caractérisé en ce qu'**au moins une partie des générateurs de haute fréquence possédant des fréquences différentes délivrent une énergie à haute fréquence.
